(19) Europäisches Patentamt
European Patent Office
Office européen des brevets

(11) Publication number: **0 345 056 B1**

(12) # EUROPEAN PATENT SPECIFICATION

(45) Date of publication of patent specification: **01.02.95**

(51) Int. Cl.[6]: **C07C 215/08**, C07D 209/08, C07C 213/04, A61K 31/135, A61K 31/40

(21) Application number: **89305516.0**

(22) Date of filing: **01.06.89**

(54) **Improvements in and relating to serotonin antagonists.**

(30) Priority: **03.06.88 US 202767**

(43) Date of publication of application:
**06.12.89 Bulletin 89/49**

(45) Publication of the grant of the patent:
**01.02.95 Bulletin 95/05**

(84) Designated Contracting States:
**AT BE CH DE ES FR GB GR IT LI LU NL SE**

(56) References cited:
**EP-A- 0 338 877**
**CS-A- 170 664**
**US-A- 3 471 515**
**US-A- 3 551 493**
**ZA-A- 687 915**

**ZBORNIK PRAC USTAVU EXPERIMENTALNEJ FARMAKOLOGIE SAV, vol. 1, 1978, pages 85-96; J. DRIMAL et al.: "Intensive beta-adrenolytic activity of cyclohexyl-derivatives of aryloxyamino-propanols /C-APA/"**

(73) Proprietor: **ELI LILLY AND COMPANY**
**Lilly Corporate Center**
**Indianapolis**
**Indiana 46285 (US)**

(72) Inventor: **Beedle, Edward Earl**
**4119 Sherlock Drive**
**Indianapolis**
**Indiana 46254 (US)**
Inventor: **Robertson, David Wayne**
**4290 Hunters Ridge Lane**
**Greenwood**
**Indiana 46142 (US)**
Inventor: **Wong, David Taiwai**
**1640 Ridge Hill Lane**
**Indianapolis**
**Indiana 46217 (US)**

(74) Representative: **Hudson, Christopher Mark et al**
**Lilly Industries Limited**
**European Patent Operations**
**Erl Wood Manor**
**Windlesham**
**Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 345 056 B1

J. MED. CHEM., vol. 11, 1968, pages 1009-1013; A.F. CROWTHER et al.: "Beta-adrenergic blocking agents. II. Propranolol and related 3-amino-1-naphthoxy-2-propanols"

ANNUAL REPORTS IN MEDICINAL CHEMISTRY, vol. 21, 1986, pages 41-50, Academic Press, Inc.; D.N. MIDDLEMISS et al.: "Drugs acting at central 5-hydroxytryptamine receptors"

TIPS, vol. 7, no. 9, September 1986, pages 336-338, Elsevier Science Publishers B.V., Amsterdam, NL; M. HAMON et al.: "Are there selective ligands for 5-HT1a and 5-HT1b receptor binding sites in brain?"

IDEM

## Description

This invention provides compounds of the propanolamine class having selective 5-$HT_1$ activity with minimal effects upon the $\beta$-receptor.

A variety of compounds are known that affect serotonin receptors. However, only recently has it been appreciated that several recognition sites and subtypes of the serotonin receptor exist -- see generally Middlemiss, Annual Reports of Medicinal Chemistry, 21, 41-50 (Academic Press, 1986) and Glennon, J. Med. Chem., 30(1), 1-12 (1987). For example, compounds known to be selective ligands for the 5-$HT_1$ receptor have been shown to affect the cardiovascular system, feeding behavior, sexual activity, gastrointestinal function, and body temperature.

A variety of propanolamine compounds are disclosed in the prior art, for example, in ZA-A- 687 915, CS-A- 170 664, Zbornik Prac Ustavu Experimentalnej Farmakologie Sav, Vol. I, 1978, pages 85-96, US-A-3 551 493, J. Med. Chem., vol 11, 1968, pages 1009-1013 and Annual Reports in Medicinal Chemistry, Vol. 21, 1986, pages 41-50.

Several propanolamines, such as pindolol and propranolol, have been shown to be 5-$HT_1$ antagonists. However, they are not selective agents for the serotonin receptor due to their $\beta$-blocking activities.

This invention provides the use of a compound in the manufacture of a medicament for selectively antagonizing the 5-$HT_1$ receptor in a mammal, the compound having the Formula I

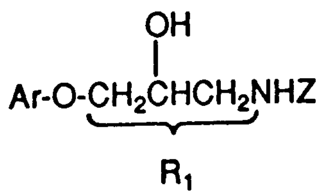

I

where Ar is

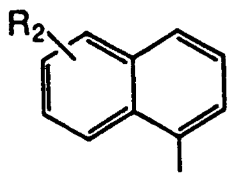

,

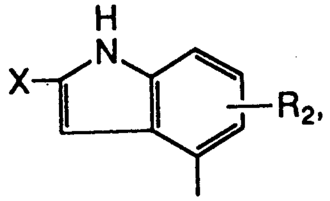

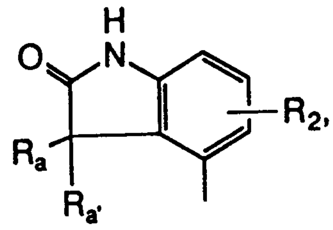

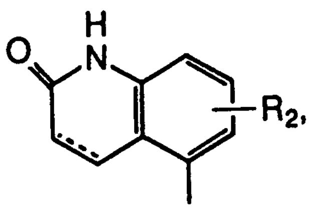

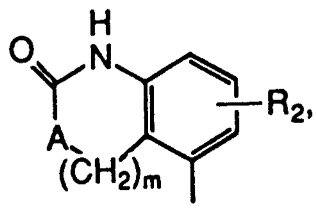

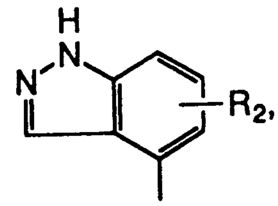

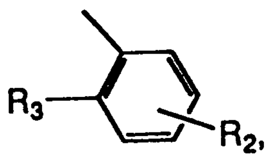

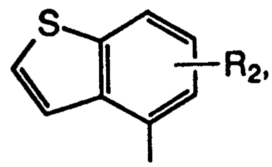

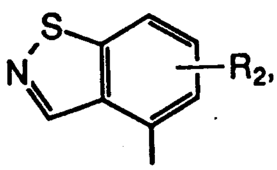

or

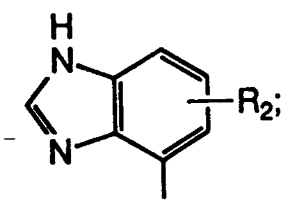

$R_1$ is an optional methyl group substituted on one of the three connecting carbon atoms;
$R_2$ is hydrogen, $C_1$-$C_4$ alkyl, trifluoromethyl, hydroxy, ($C_1$-$C_4$ alkyl)-O-, ($C_1$-$C_4$ alkyl)-S(O)$_p$-, or halo;
$R_3$ is $C_3$-$C_8$ cycloalkyl or a bicycloalkyl group of the formula

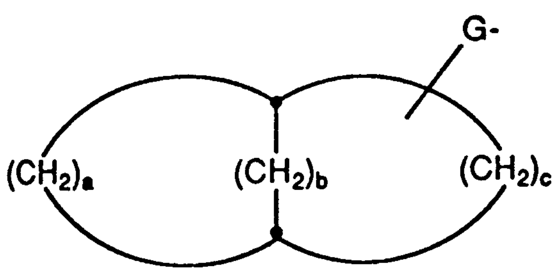

,

where a and c are independently 1-5, b is 0-5, and (a + c) is greater than 2;
Z is a straight or branched $C_4$-$C_{10}$ alkane, alkene, or alkyne group, ($C_4$-$C_8$ cycloalkyl)-G- optionally substituted with $C_1$-$C_4$ alkyl or phenyl, a bicycloalkyl group of the formula

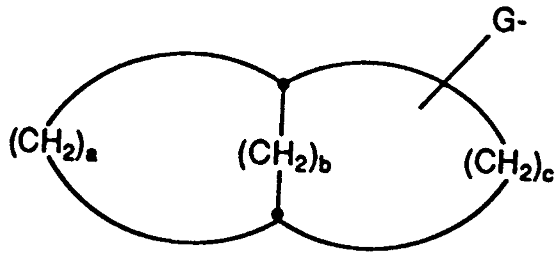

wherein a and c are independently 1-5, b is 0-5, and (a + c) is greater than 2, optionally phenyl substituted $C_2$-$C_{10}$ alkyl where the phenyl group can be optionally substituted with $R_2$ as previously defined, or -($C_1$-$C_4$ alkylidene)-T-($C_1$-$C_4$ alkyl), where T is -O-, -S-, -SO-, or $SO_2$-;

where

each G is independently a bond or $C_1$-$C_4$ alkylidene;

X is -H or $C_1$-$C_4$ alkyl;

---- represents a single or double bond;

$R_a$ and $R_{a'}$ are independently hydrogen or $C_1$-$C_3$ alkyl, or when taken together with the carbon atom to which they are attached form a $C_3$-$C_8$ cycloalkyl ring;

p is 0, 1, or 2;

A is -O-, -S-, -NH-, or -$NCH_3$-; and

m is 0, 1, 2, or 3;

or a pharmaceutically-acceptable salt thereof.

Although it is known that compounds such as pindolol and propranolol antagonize the 5-$HT_{1A}$ and 5-$HT_{1B}$ receptors, they do so at concentrations far in excess of those shown to block the $\beta$-receptor. Accordingly, their usefulness in treating disease states and conditions related to the 5-$HT_1$ receptors are limited.

We have discovered the unexpected finding that extending or otherwise substituting the isopropylamine substituent of such compounds with a longer and/or more sterically hindered substituent on the amine function provides compounds which are much more selective antagonists of the 5-$HT_1$ receptor, i.e., the ratio of concentrations to antagonize the $\beta$-receptor compared with the 5-$HT_{1A}$ receptor is at least 1.0. Accordingly, the compounds employed in the present invention should be useful for treating a variety of conditions related to the 5-$HT_1$ receptor without the concomitant effects seen with $\beta$-blockers. The desired effects seen with the compounds employed in the present invention therefore include treatment of sexual dysfunction, depression, appetite disorders, anxiety, schizophrenia, gastrointestinal disorders, headaches, and cardiovascular disorders.

The term "$C_1$-$C_4$ alkyl" refers to methyl, ethyl, propyl, isopropyl, butyl, sec-butyl, t-butyl, and isobutyl. The term "$C_4$-$C_8$ cycloalkyl" refers to cyclobutyl, cyclopentyl, cyclohexyl, cycloheptyl, and cyclooctyl. Optional substituents on the $C_5$-$C_8$ cycloalkylring maybe at any position or orientation on the ring other than at the point of attachment to the nitrogen atom.

The term "straight or branched $C_4$-$C_{10}$ alkane" includes alkyl groups of 4-10 carbon atoms as either straight-chain hydrocarbons or with one or more points of branching. Similarly, the term "straight or branched $C_4$-$C_{10}$ alkene or alkyne group" refers to similar straight or branched hydrocarbon chains containing a double or triple bond, respectively. "Halo" refers to fluoro, chloro, bromo, and iodo.

The term "-($C_1$-$C_4$ alkylidene)-T-($C_1$-$C_4$ alkyl)" refers to two straight or branched $C_1$-$C_4$ alkyl groups bridged by the T functionality. The term "$C_1$-$C_4$ alkylidene" refers to a divalent radical derived from a $C_1$-$C_4$ alkane.

The bicycloalkyl groups defined as part of the $R_3$ and Z substituents include bicyclic rings of four to seventeen carbon atoms. These bicycloalkyl groups include bridged and fused two-ring systems. Examples of representative Z substituents are provided in Table I.

The $R_1$ optional methyl group is one wherein the three-carbon bridge between the aryloxy and amine functionalities are optionally substituted with a methyl group. That is, in addition to the -$CH_2CH(OH)CH_2$- bridge as drawn in Figure I, such bridging groups also include -$CH(CH_3)CH(OH)CH_2$-, -$CH_2C(OH)(CH_3)CH_2$-, and -$CH_2CH(OH)CH(CH_3)$-.

It is recognized that depending upon the $R_1$, hydroxy, and Z substituent groups, one or more steroisomers and/or enantiomers are possible. This invention is not limited to any particular isomer but includes all possible individual isomers and all combinations thereof.

The pharmaceutically acceptable addition salts employed in this invention include salts derived from inorganic acids such as hydrochloric acid, nitric acid, phosphoric acid, sulfuric acid, hydrobromic acid, hydriodic acid, phosphorus acid, and the like, as well as salts derived from organic acids, such as aliphatic mono- or di-carboxylic acids, phenyl-substituted alkanoic acids, hydroxy-alkanoic and -alkandioic acids, aromatic acids, aliphatic and aromatic sulfonic acids, and the like.

The preferred compounds employed in this invention are those of the propranolol (Ar = 1-naphthyl) and especially the pindolol (Ar = 4-indolyl), cyanopindolol (Ar = 2-cyano-4-indolyl), and most especially penbutolol (Ar = 2-cyclopentylphenyl) type. The preferred $R_1$ group is hydrogen, and preferred Z substituents are branched alkyl groups, particularly those of 6-8 carbon atoms, and cyclohexyl, cycloheptyl, cyclooctyl, and bicycloalkyl derivatives.

The compounds employed in the present invention are antagonists of the serotonin 5-$HT_1$ receptor and have minimal effects upon the $\beta$-receptor. The compounds of this invention were evaluated in the following tests systems.

Procedure for radioligand receptor assays --Male Sprague-Dawley rats weighing 110-150 g were decapitated and brains were immediately removed. Bovine brains were quickly removed from calf in the slaughter-house. Cerebral cortices were dissected out at 4°C and homogenized in 0.32 M sucrose. After centrifugation at 1000 x g for 10 minutes and then 17,000 x g for 20 minutes, a crude synaptosomal fraction was sedimented. The pellet was suspended in 100 volumes of 50 mM tris-HCl buffered at pH 7.4, incubated at 37°C for 10 minutes, and centrifuged at 50,000 x g for 10 minutes. The process was repeated and the final pellet was suspended in ice-chilled 50 mM tris-HCl buffered at pH 7.4.

Binding of $^3$H-8-hydroxy-2-(di-n-propylamino)tetralin(3H-8OHDPAT) to serotonin-1A receptor was performed according to the method described by Wong et al., J. Neural Transm., 71, 207 (1988). Cortical membranes (300 $\mu$g protein) isolated from rat brain were incubated at 28 C for 30 minutes in 2 ml of medium containing 50 mM tris-HCl buffered at pH 7.4; 10 $\mu$M pargyline; 0.6 mM ascorbic acid; 5 mM calcium chloride; 0.4 nM 3H-8OHDPAT; and various concentrations of test compound. Binding was terminated by filtering samples under reduced pressure through glass fiber (GFB) filters. The filters were washed twice with 5 ml of ice cold buffer and placed in scintillation vials with 10 ml of PCS (Amersham/Searle) scintillation fluid. Radioactivity was measured with a liquid scintillation spectrometer. Specific binding of 3H-8OHDPAT is defined as the difference of radioactivity bound in the absence and in the presence of 10 $\mu$M spiperone. Concentration of compound required to cause a 50% inhibition ($IC_{50}$ value) of 3H-8OHDPAT binding was computed by linear regression analysis and is reported in Table I as the 5-$HT_{1A}$ $IC_{50}$.

Binding of 3H-dihydroalprenolol to $\beta$-adrenergic receptors was conducted according to the method of Wong et al., Biochemical Pharmacology, 32(7), 1287 (1983). Cortical membranes (500 $\mu$g protein) of calf brain were incubated at 20°C for 20 minutes in 2 ml of medium containing 50 mM tris-HCl buffered at pH 7.4; 2 nM 3H-dihydroalprenolol; and various concentrations of test compound. Binding was terminated by filtering samples through glass fiber (GFC) filters. L-propranolol at 10 $\mu$M was included in separate samples to establish nonspecific binding. Other conditions were as described for 3H-8OHDPAT binding. Table I reports the results of this testing as the beta $IC_{50}$.

Illustrative compounds of this invention as evaluated in these test systems are reported in Tables I, II, and III. For comparative purposes, the test data for propranolol, i.e., the analogous compound wherein Z is isopropyl, is provided. Also provided is the ratio of the respective $IC_{50}$s.

TABLE I

Effect of Compounds of Formula I to Antagonize the $5\text{-}HT_{1A}$ and Beta Receptors

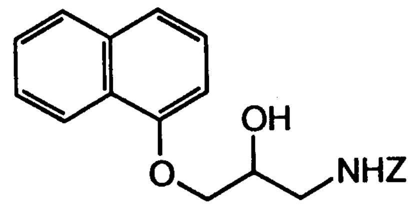

| Z | $IC_{50}$ (nM) $5\text{-}HT_{1A}$ | $IC_{50}$ (nM) Beta | Ratio Beta $IC_{50}$/ $5\text{-}HT_{1A}$ $IC_{50}$ |
|---|---|---|---|
| $-C(CH_3)_2CH{=}CH_2$ | 100 | 124 | 1.24 |
| $-CH(CH_3)C{\equiv}CH$ | 180 | 1270 | 7.1 |
| $-C(CH_3)_2C{\equiv}CCH_3$ | 38 | 398 | 10.5 |
| $-C(CH_3)_2C{\equiv}CH$ | 88 | 1358 | 15.4 |
| $-C(CH_3)_2CH_2CH_3$ | 53 | 1046 | 20 |
| $-CH_2CH_2CH_2CH_3$ | 36.9 | 124 | 3.4 |
| $-CH_2CH_2CH_2CH_2CH_3$ | 23.4 | 185 | 7.9 |
| $-CH_2CH(CH_3)_2$ | 73 | 683 | 9.4 |
| $-C(CH_3)_2C(CH_3)_3$ | 16.8 | 225 | 15.2 |

TABLE I (Continued)

| Z | $IC_{50}$ (nM) 5-$HT_{1A}$ | Beta | Ratio Beta $IC_{50}$/ 5-$HT_{1A}$ $IC_{50}$ |
|---|---|---|---|
| -(cyclohexyl) | 15.4 | 622 | 40.4 |
| $-CH(CH_3)C(CH_3)_3$ | 152.3 | 1205 | 7.9 |
| $-C(CH_3)_2CH_2CH_2CH_3$ | 41 | 98 | 2.4 |
| -(2-methylcyclohexyl) | 33.4 | 174 | 5.2 |
| $-CH(CH_3)CH_2CH_3$ | 73 | 104 | 1.4 |
| -(E-4-phenylcyclohexyl) | >1000 | 264 | <0.26 |
| -(Z-4-phenylcyclohexyl) | 34.1 | 442 | 13 |
| -(cyclooctyl) | 45 | 1000 | 22.2 |
| -(cyclopentyl) | 49 | 138 | 2.8 |
| -(cycloheptyl) | 23 | 2285 | 99 |
| $-C(CH_3)_3$ | 77 | 97.3 | 1.3 |
| $-CH(CH_3)CH_2OCH_3$ | 147 | 65 | 2.3 |
| $-CH_2CH_2C_6H_5$ | 63 | 313 | 4.8 |
| $-CH(CH_3)_2$ (propranolol) | 100 | 5 | 0.05 |

## TABLE II

Effect of Compounds of Formula I to Antagonize the 5-$HT_{1A}$ and Beta Receptors

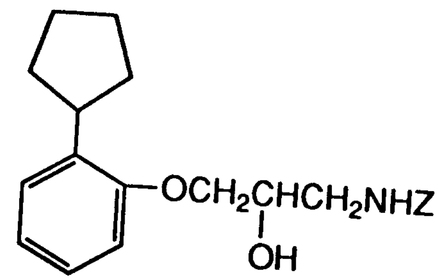

| Z | $IC_{50}$ (nM) 5-$HT_{1A}$ | $IC_{50}$ (nM) Beta | Ratio Beta $IC_{50}$/ 5-$HT_{1A}$ $IC_{50}$ |
|---|---|---|---|
| cycloheptyl | 3 | 48 | 16 |
| cyclooctyl | 5 | >1000 | >200 |
| cyclopentyl | 5 | 7 | 1.4 |
| $CH(CH_3)C(CH_3)_3$ | 13 | 61 | 4.7 |
| $CH(CH_3)_2CH_2CH_2CH_3$ | 6.5 | 16 | 2.5 |

TABLE III

Effect of Compounds of Formula I to Antagonize the $5\text{-}HT_{1A}$ and Beta Receptors

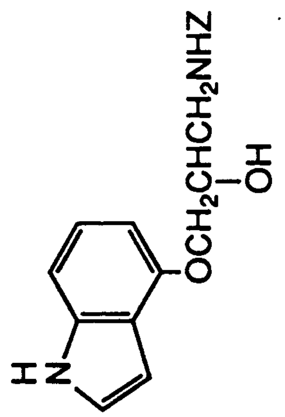

| Z | $IC_{50}$ (nM) $5\text{-}HT_{1A}$ | $IC_{50}$ (nM) Beta | Ratio Beta $IC_{50}$/ $5\text{-}HT_{1A}$ $IC_{50}$ |
|---|---|---|---|
| Cyclohexyl | 10.3 | 56 | 5.4 |

As can be discerned from the above test results, those derivatives having longer and/or more branched amino substituents tend to have greater affinity for the $5\text{-}HT_{1A}$ receptor and have increasing selectivity over the $\beta$-receptor. For example, in the cycloalkyl series, increasing the ring size from 5 to 6 to 7 carbon atoms results in an increasing ratio of 2.8, 40.4, and 99, respectively. Similarly, those alicyclic substituents which have multiple branching, i.e., contained a plurality of methyl groups off of a straight hydrocarbon, are more potent and have greater selectivity than their non-branched congeners. It is interesting to note that the sec-butyl and isobutyl derivatives described above are 28 and almost 200 times more selective, respectively, than propranolol which is an isopropyl derivative.

Because of their selective affinity for the $5\text{-}HT_{1A}$ receptor, the compounds employed in the present invention should be useful for treating a variety of conditions such as obesity, bulemia, depression, hypertension, aging, memory loss, sexual dysfunction, anxiety, schizophrenia, gastrointestinal disorders, headaches and cardiovascular disorders. To treat such conditions, a typical daily dose will contain a

pharmaceutically effective amount of one of the compounds of Formula I. The term "pharmaceutically amount", as used herein, represents an amount of a compound of Formula I which is capable of affecting the 5-$HT_{1A}$ receptor in a mammal. The particular dose of a compound administered will, of course, be determined by the particular circumstances surrounding the use, including the specific compound administered, the route of administration, the particular condition being treated, and similar conditions. A typical daily dose will contain from about 0.01 mg/kg to about 20 mg/kg of a compound of Formula I.

The compounds can be administered by a variety of routes including the oral, rectal, transdermal, subcutaneous, intravenous, intramuscular, or intranasal routes. The compounds can be administered in pharmaceutical formulations comprising a compound of Formula I together with one or more pharmaceutically acceptable excipients, diluents, or carriers therefor. Such formulations are well known in this art and can be adapted to provide a unit dosage form depending on the route of administration and other factors as mentioned above.

This invention also provides novel compounds of the above types. The compounds are represented by Formula Ia

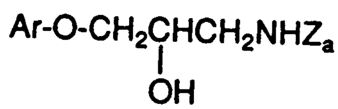

Ia

where $Z_a$ is ($C_6$-$C_8$ cycloalkyl)-G-, $C_6$-$C_{10}$ alkyl, phenylsubstituted $C_2$-$C_{10}$ alkyl, -($C_1$-$C_4$ alkylidene)-T-($C_1$-$C_4$ alkyl), or

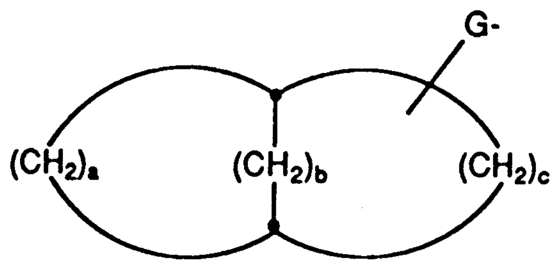

and Ar is

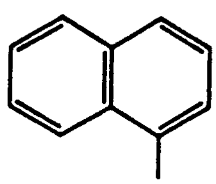

or

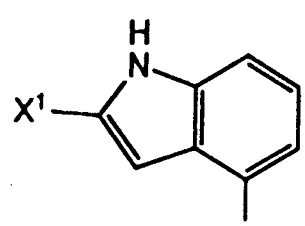

where $X^1$ is -H or -CN and G, T, a, b and c are the same as previously defined, and pharmaceutically acceptable salts thereof, provided

a) when Ar is 1-naphthyl, $Z_a$ may not be cyclohexyl, $C_6$-$C_{10}$ alkyl, phenylsubstituted $C_2$-$C_{10}$ alkyl, or -($C_1$-$C_4$ alkylidene)-T-($C_1$-$C_4$ alkyl); and

b) when Ar is

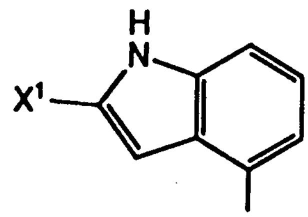

$Z_a$ may not be phenyl substituted $C_2$-$C_{10}$ alkyl; ($C_1$-$C_4$ alkylidene)-T-($C_1$-$C_4$ alkyl), or

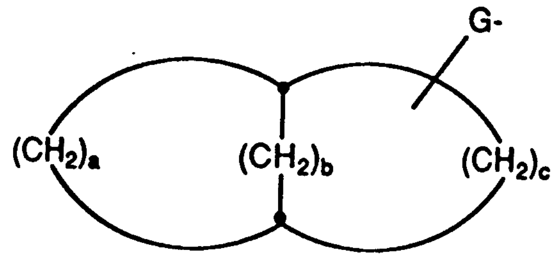

The compounds employed in the method of this invention are known in the art or can be prepared by methods known in the art. References illustrative of this chemistry include Crowther et al., J. Med. Chem., 11, 1009 (1968) (propranolol and derivatives), U.S. Patent 3,551,493 (penbutolol and derivatives), and U.S. Patent 3,471,515 (pindolol and derivatives), which references are expressly incorporated within this application.

A further aspect of this invention involves a process for preparing the novel compounds of this invention. In general, the compounds are best prepared according to the following scheme:

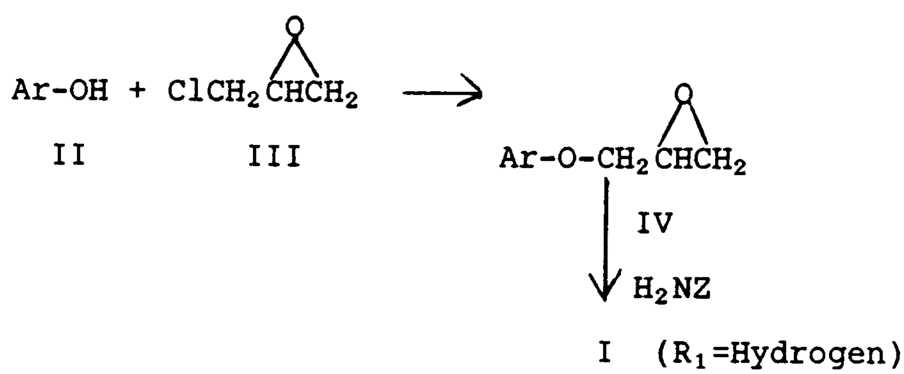


According to Scheme I, the hydroxy aromatic intermediate II is reacted with epichlorohydrin (III) or a halo congener thereof to provide the epoxy derivative IV. This chemistry is well established in the art and can be conducted in a number of ways, generally employing an inert solvent and a nonreactive acid scavenger Epoxide IV is then reacted with the primary amine $H_2NZ$ to provide the compounds employed in this invention. Once again, the reaction is usually performed in the presence of a nonreactive solvent and at elevated temperatures up to the reflux temperature of the reaction mixture. Scheme I is drawn contemplating those compounds wherein $R_1$ is hydrogen; by employing the appropriately substituted halo epoxide derivative III, the other compounds employed in the present invention may be prepared in a similar manner. The pharmaceutically acceptable salts optionally employed in the present invention may also be prepared by standard methods known to those skilled in this art.

The following Examples are illustrative of the novel and/or of particularly preferred compounds for the use in the manufacture of a medicament of this invention.

Example 1

1-(1-Naphthalenoxy)-3-(cyclooctylamino)-2-propanol ethanedioate

A mixture of 3.17 g of 3-(1-naphlhalenoxy)-1,2-epoxypropane and 2.01 g of cyclooctylamine were heated to reflux in methanol overnight. The mixture was cooled, concentrated in vacuo, diluted with ethyl acetate and treated with a solution of oxalic acid in ethyl acetate. The resulting precipitate was recovered by filtration and crystallized from ethyl acetate/diethyl ether to provide 4.3 g of the title product, m.p. 147-148 °C.

The following compounds were prepared in similar fashion from the appropriate aryloxy-epoxypropane and corresponding amine:

1-(1-Naphthalenoxy)-3-(cycloheptylamino)-2-propanol ethanedioate, 47% yield, m.p. 161-162 °C,
1-(4-Indolyloxy)-3-(endo-2-norbornanylamino)-2-propanol ethanedioate,
1-(2-Cyclopentylphenoxy)-3-(cycloheptylamino)-2-propanol ethanedioate, 47% yield, m.p. 192-194 °C,
1-(2-Cyclopentylphenoxy)-3-(cyclooctylamino)-2-propanol ethanedioate, 10% yield,
1-(2-Cyclopentylphenoxy)-3-(1,2,2-dimethyl-2-propylamino)-propanol ethanedioate, 9% yield,
1-(2-Cyclopentylphenoxy)-3-(1,1-dimethylbutylamino)-2-propanol ethanedioate, 21% yield,
1-(2-Cyclopentylphenoxy)-3-(myrtanylamino)-2-propanol ethanedioate,
1-(4-Indolyloxy)-3-(cyclohexylamino)-2-propanol maleate, 55% yield, m.p. 134-135 °C,
1-(2-Cyclopentylphenoxy)-3-cyclohexylamino-2-propanol ethanedioate, 77% yield, m.p. 214-215 °C.

One further aspect of this invention provides pharmaceutical compositions comprising a compound of Formula Ia and one or more pharmaceutically acceptable excipients, diluents, or carriers therefor.

The compounds or formulations of the present invention may be administered by the oral and rectal routes, topically, parenterally, e g., by injection and by continuous or discontinuous intra-arterial infusion, in the form of, for example, tablets, lozenges, sublingual tablets, sachets, cachets, elixirs, gels, suspensions, aerosols, ointments, for example, containing from 1 to 10% by weight of the active compound in a suitable base, soft and hard gelatin capsules, suppositories, injectable solutions and suspensions in physiologically acceptable media, and sterile packaged powders adsorbed onto a support material for making injectable solutions. Advantageously for this purpose, compositions may be provided in dosage unit form, preferably each dosage unit containing from about 5 to about 500 mg (from about 5 to 50 mg in the case of parenteral or inhalation administration, and from about 25 to 500 mg in the case of oral or rectal administration) of a compound of Formula Ia. Dosages of from about 0.5 to about 300 mg/kg per day, preferably 0.5 to 20 mg/kg, of active ingredient may be administered although it will, of course, readily be understood that the amount of the compound or compounds of Formula Ia actually to be administered will be determined by a physician, in the light of all the relevant circumstances including the condition to be treated, the choice of compound to be administered and the choice of route of administration and therefore the above preferred dosage range is not intended to limit the scope of the present invention in any way.

The formulations of the present invention normally will consist of at least one compound of Formula Ia mixed with a carrier, or diluted by a carrier, or enclosed or encapsulated by an ingestible carrier in the form of a capsule, sachet, cachet, paper or other container or by a disposable container such as an ampoule. A carrier or diluent may be a solid, semi-solid or liquid material which serves as a vehicle, excipient or medium for the active therapeutic substance.

Some examples of the diluents or carrier which may be employed in the pharmaceutical compositions of the present invention are lactose, dextrose, sucrose, sorbitol, mannitol, propylene glycol, liquid paraffin, white soft paraffin, kaolin, fumed silicon dioxide, microcrystalline cellulose, calcium silicate, silica, polyvinylpyrrolidone, cetostearyl alcohol, starch, modified starches, gum acacia, calcium phosphate, cocoa butter, ethoxylated esters, oil of theobroma, arachis oil, alginates, tragacanth, gelatin, syrup, methyl cellulose, polyoxyethylene sorbitan monolaurate, ethyl lactate, methyl and propyl hydroxybenzoate, sorbitan trioleate, sorbitan sesquioleate and oleyl alcohol and propellants such as trichloromonofluoromethane, dichlorodifluoromethane and dichlorotetrafluoroethane. In the case of tablets, a lubrioant may be incorporated to prevent sticking and binding of the powdered ingredients in the dies and on the punch of the tableting machine. For such purpose there may be employed for instance aluminum, magnesium or calcium stearates, talc or mineral oil.

Preferred pharmaceutical forms of the present invention are capsules, tablets, suppositories, injectable solutions, creams and ointments. Especially preferred are formulations for inhalation application, such as an aerosol, and for oral ingestion.

The following formulation examples may employ as active compounds any of the compounds of this invention. The examples are illustrative only and are not intended to limit the scope of the invention in any

way.

Example 2

Hard gelatin capsules are prepared using the following ingredients:

| | Quantity (mg/capsule) |
|---|---|
| 1-(1-Naphthalenoxy)-3-(cyclooctylamino)-2-propanol ethanedioate | 250 |
| Starch | 200 |
| Magnesium stearate | 10 |

The above ingredients are mixed and filled into hard gelatin capsules in 460 mg quantities.

Example 3

A tablet is prepared using the ingredients below:

| | Quantity (mg/tablet) |
|---|---|
| 1-(1-Naphthalenoxy)-3-(cycloheptylamino)-2-propanol ethanedioate | 250 |
| Cellulose, microcrystalline | 400 |
| Silicon dioxide, fumed | 10 |
| Magnesium stearate | 5 |

The components are blended and compressed to form tablets each weighing 665 mg.

Example 4

An aerosol solution is prepared containing the following components:

| | Weight % |
|---|---|
| 1-(2-Cyclopentylphenoxy)-3-(cycloheptylamino)-2-propanol ethanedioate | 0.25 |
| Ethanol | 30.00 |
| Propellant 11 (trichlorofluoromethane) | 10.25 |
| Propellant 12 (Dichlorodifluoromethane) | 29.75 |
| Propellant 114 (Dichlorotetrafluoroethane) | 29.75 |

The active compound is dissolved in the ethanol and the solution is added to the propellant 11, cooled to -30 °C. and transferred to a filling device. The required amount is then fed to a container and further filled with the pre-mixed propellants 12 and 114 by means of the cold-filled method or pressure-filled method. The valve units are then fitted to the container.

Example 5

Tablets each containing 60 mg of active ingredient are made up as follows:

| | |
|---|---|
| 1-(2-Cyclopentylphenoxy)-3-(1,2,2-dimethyl-2-propylamino)-propanol ethanedioate | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50-60 °C. and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Example 6

Capsules each containing 80 mg of medicament are made as follows:

| | |
|---|---|
| 1-(4-Indolyloxy)-3-(endo-2-norbornanylamino)-2-propanol ethanedioate | 80 mg |
| Starch | 59 mg |
| Microcrystalline cellulose | 59 mg |
| Magnesium stearate | 2 mg |
| Total | 200 mg |

The active ingredient, cellulose, starch and magnesium stearate are blended, passed through a No. 45 mesh U.S. sieve, and filled into hard gelatin capsules in 200 mg quantities.

Example 7

Suppositories each containing 225 mg of active ingredient are made as follows:

| | |
|---|---|
| 1-(2-Cyclopentylphenoxy)-3-cyclohexylamino-2-propanol ethanedioate | 225 mg |
| Unsaturated or saturated fatty acid glycerides to | 2,000 mg |

The active ingredient is passed through a No. 60 mesh U.S. sieve and suspended in the fatty acid glycerides previously melted using the minimum heat necessary. The mixture is then poured into a suppository mold of nominal 2 g capacity and allowed to cool.

Example 8

Suspensions each containing 50 mg of medicament per 5 ml dose are made as follows:

| | |
|---|---|
| 1-(2-Cyclopentylphenoxy)-3-cyclohexylamino-2-propanol ethanedioate | 50 mg |
| Sodium carboxymethyl cellulose | 50 mg |
| Sugar | 1 g |
| Methyl paraben | 0.05 mg |
| Propyl paraben | 0.03 mg |
| Flavor | q.v. |
| Color | q.v. |
| Purified water to | 5 ml |

## Claims

**Claims for the following Contracting States : AT, BE, CH, DE, FR, GB, IT, LI, LU, NL, SE**

**1.** The use of a compound of the formula

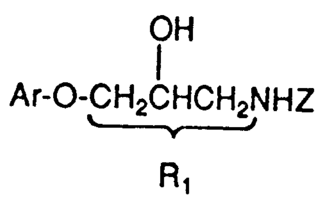

I

where Ar is

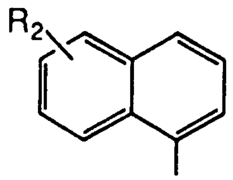
,
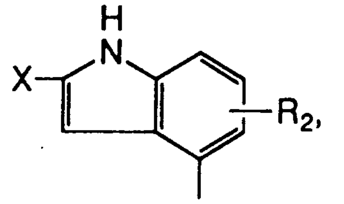
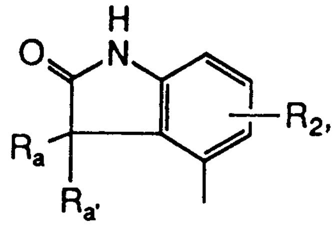
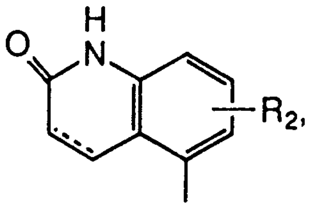
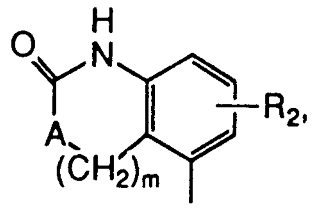
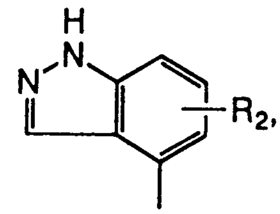
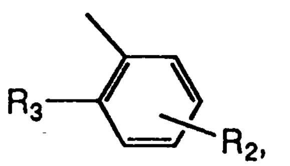
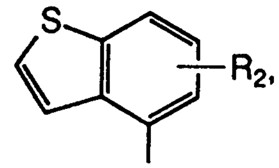
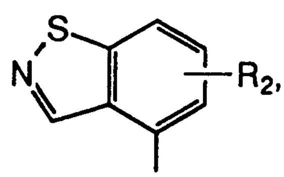
or
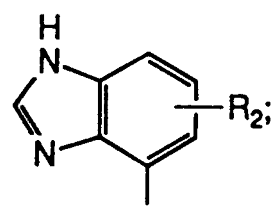

$R_1$ is an optional methyl group substituted on one of the three connecting carbon atoms;
$R_2$ is hydrogen, $C_1$-$C_4$ alkyl, trifluoromethyl, hydroxy, ($C_1$-$C_4$ alkyl)-O-, ($C_1$-$C_4$ alkyl)-S(O)$_p$-, or halo;
$R_3$ is $C_3$-$C_8$ cycloalkyl or a bicycloalkyl group of the formula

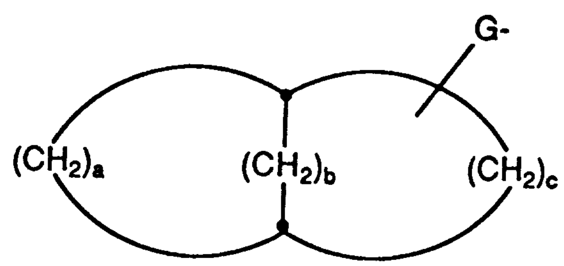
,

where a and c are independently 1-5, b is 0-5, and (a + c) is greater than 2;
Z is a straight or branched $C_4$-$C_{10}$ alkane, alkene, or
alkyne group, ($C_4$-$C_8$ cycloalkyl)-G- optionally substituted with $C_1$-$C_4$ alkyl or phenyl, a bicycloalkyl group of the formula

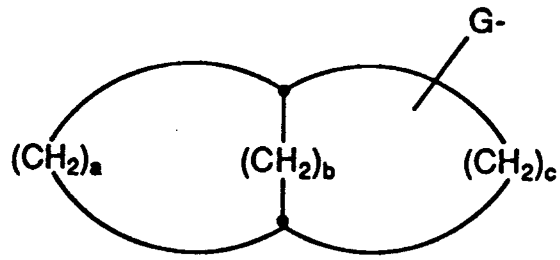

,

wherein a and c are independently 1-5, b is 0-5, and (a + c) is greater than 2, optionally phenyl substituted $C_2$-$C_{10}$ alkyl where the phenyl group can be optionally substituted with $R_2$ as previously defined, or -($C_1$-$C_4$ alkylidene)-T-($C_1$-$C_4$ alkyl), where T is -O-, -S-, -SO-, or -$SO_2$-;

where

each G is independently a bond or $C_1$-$C_4$ alkylidene;

X is -H or $C_1$-$C_4$ alkyl;

---- represents a single or double bond;

$R_a$ and $R_{a'}$ are independently hydrogen or $C_1$-$C_3$ alkyl, or when taken together with the carbon atom to which they are attached form a $C_3$-$C_8$ cycloalkyl ring;

p is 0, 1, or 2;

A is -O-, -S-, -NH-, or -$NCH_3$-; and

m is 0, 1, 2, or 3;

or a pharmaceutically-acceptable salt thereof, in the manufacture of a medicament for selectively antagonizing the 5-$HT_1$ receptor in a mammal.

2. The use according to Claim 1 employing a compound wherein Ar is 1-naphthyl, 4-indolyl, or 2-cyclopentylphenyl.

3. The use according to Claim 1 employing a compound wherein Z is cyclohexyl, cycloheptyl, cyclooctyl, or $C_6$-$C_8$ alkyl.

4. The use according to Claim 2 employing 1-(2-cyclopentylphenoxy)-3-(cycloheptylamino)-2-propanol or a pharmaceutically acceptable salt thereof.

5. The use according to Claim 2 employing 1-(2-cyclopentylphenoxy)-3-(cyclooctylamino)-2-propanol or a pharmaceutically acceptable salt thereof.

6. A compound of the Formula Ia

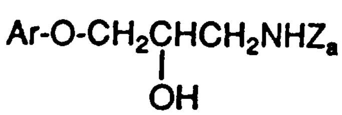

Ia

where $Z_a$ is ($C_6$-$C_8$ cycloalkyl)-G-, $C_6$-$C_{10}$ alkyl, phenylsubstituted $C_2$-$C_{10}$ alkyl, -($C_1$-$C_4$ alkylidene)-T-($C_1$-$C_4$ alkyl), or

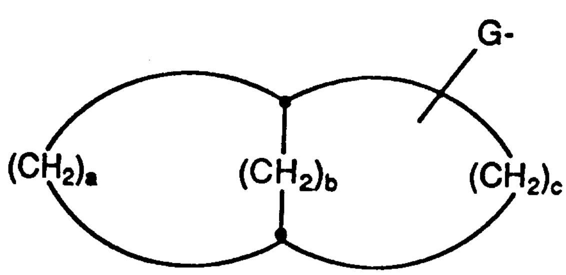

,

and Ar is

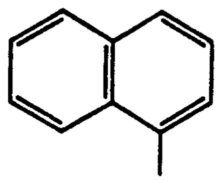

or

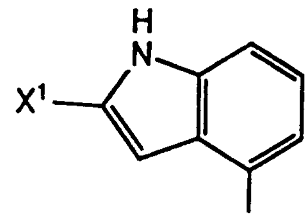

where

$X^1$ is -H or -CN;

each G is independently a bond or $C_1$-$C_4$ alkylidene;

T is -O-, -S-, -SO-, or -$SO_2$-; and

a and c are independently 1-5, b is 0-5, and (a + c) is greater than 2;

and pharmaceutically acceptable salts thereof, provided

a) when Ar is 1-naphthyl, $Z_a$ may not be cyclohexyl, $C_6$-$C_{10}$ alkyl, phenylsubstituted $C_2$-$C_{10}$ alkyl, or -($C_1$-$C_4$ alkylidene)-T-($C_1$-$C_4$ alkyl); and

b) when Ar is

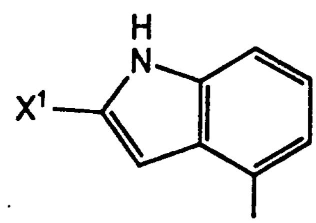

$Z_a$ may not be phenylsubstituted $C_2$-$C_{10}$ alkyl; ($C_1$-$C_4$ alkylidene)-T-($C_1$-$C_4$ alkyl), or

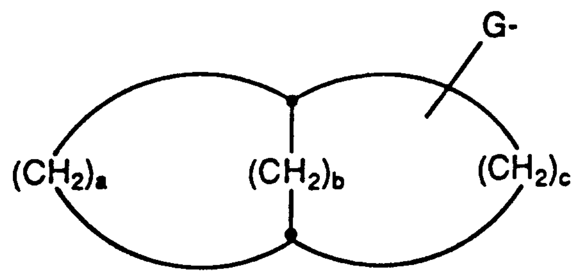

**7.** A pharmaceutical composition comprising a compound as claimed in Claim 6, in association with one or more pharmaceutically acceptable excipients, diluents, or carriers therefor.

**8.** A process for preparing a compound as claimed in Claim 6, which comprises reacting an epoxide compound of the formula

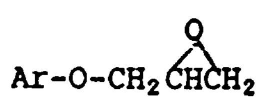

with an amine of the formula $H_2NZ_a$ and optionally converting the resulting product into a pharmaceutically acceptable salt.

**Claims for the following Contracting States : ES, GR**

1. A process for preparing a compound of the Formula Ia

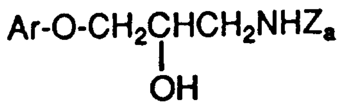

Ia

where $Z_a$ is ($C_6$-$C_8$ cycloalkyl)-G-, $C_6$-$C_{10}$ alkyl, phenylsubstituted $C_2$-$C_{10}$ alkyl, -($C_1$-$C_4$ alkylidene)-T-($C_1$-$C_4$ alkyl), or

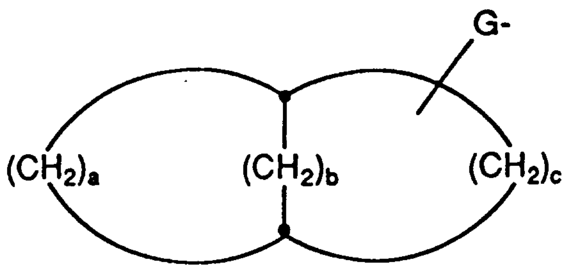

,

and Ar is

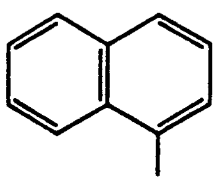

or

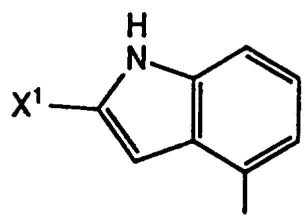

where
X[1] is -H or -CN;
each G is independently a bond or $C_1$-$C_4$ alkylidene;
T is -O-, -S-, -SO-, or -$SO_2$-; and
a and c are independently 1-5, b is 0-5, and (a + c) is greater than 2;
and pharmaceutically acceptable salts thereof, provided
a) when Ar is 1-naphthyl, $Z_a$ may not be cyclohexyl, $C_6$-$C_{10}$ alkyl, phenylsubstituted $C_2$-$C_{10}$ alkyl, or -($C_1$-$C_4$ alkylidene)-T-($C_1$-$C_4$ alkyl); and
b) when Ar is

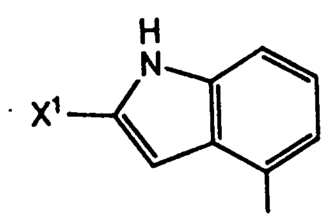

$Z_a$ may not be phenylsubstituted $C_2$-$C_{10}$ alkyl; ($C_1$-$C_4$ alkylidene)-T-($C_1$-$C_4$ alkyl), or

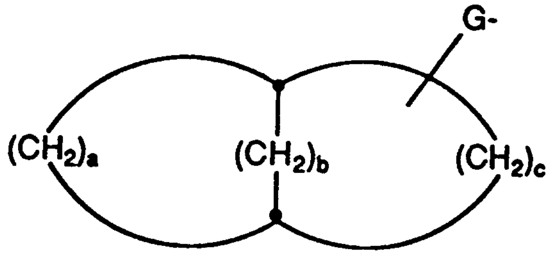

;

which process comprises reacting an epoxide compound of the formula

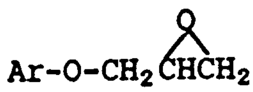

with an amine of the formula $H_2NZ_a$ and optionally converting the resulting product into a pharmaceutically acceptable salt.

2. A process according to Claim 1 for preparing a compound wherein Ar is 1-naphthyl, 4-indolyl or 2-cyano-4-indolyl.

3. A process according to Claim 1 for preparing a compound wherein $Z_a$ is cyclohexyl, cycloheptyl, cyclooctyl, or $C_6$-$C_8$ alkyl.

4. A process for preparing a pharmaceutical formulation which comprises admixing a compound of Formula Ia as defined in any one of Claims 1 to 3, or a pharmaceutically acceptable salt thereof, with one or more pharmaceutically-acceptable carriers, excipients, or diluents therefor.

5. The use of a compound of the formula

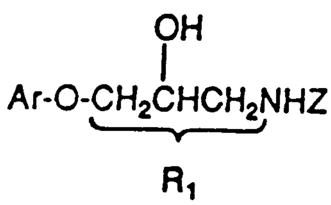

I

where Ar is

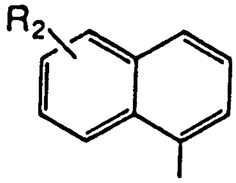

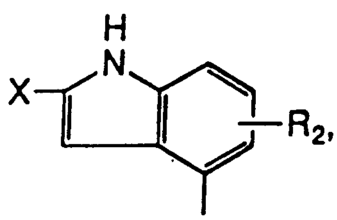

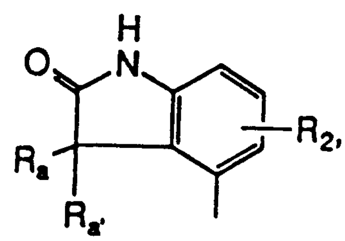

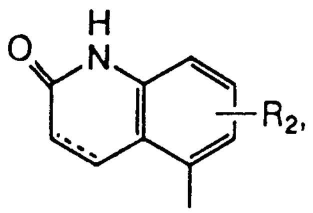, 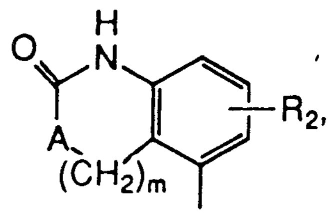 , 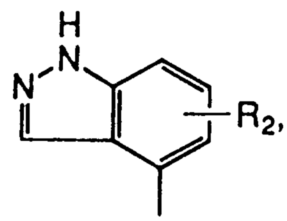

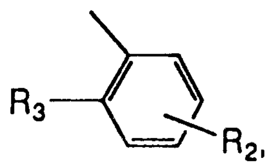 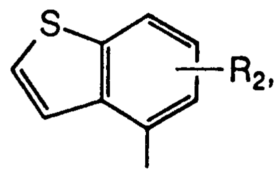

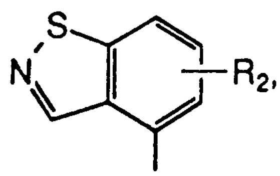 or 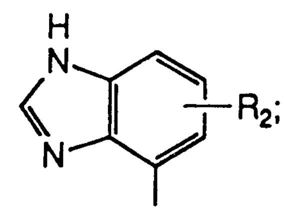

$R_1$ is an optional methyl group substituted on one of the three connecting carbon atoms;
$R_2$ is hydrogen, $C_1$-$C_4$ alkyl, trifluoromethyl, hydroxy, ($C_1$-$C_4$ alkyl)-O-, ($C_1$-$C_4$ alkyl)-S(O)$_p$-, or halo;
$R_3$ is $C_3$-$C_8$ cycloalkyl or a bicycloalkyl group of the formula

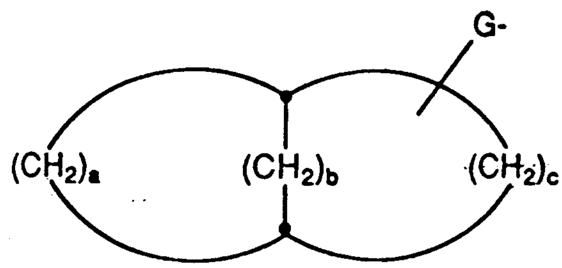

where a and c are independently 1-5, b is 0-5, and (a + c) is greater than 2;
Z is a straight or branched $C_4$-$C_{10}$ alkane, alkene, or alkyne group, ($C_4$-$C_8$ cycloalkyl)-G- optionally substituted with $C_1$-$C_4$ alkyl or phenyl, a bicycloalkyl group of the formula

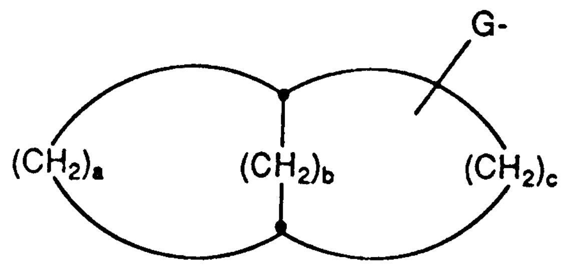 ,

wherein a and c are independently 1-5, b is 0-5, and (a + c) is greater than 2, optionally phenyl substituted $C_2$-$C_{10}$ alkyl where the phenyl group can be optionally substituted with $R_2$ as previously

defined, or -($C_1$-$C_4$ alkylidene)-T-($C_1$-$C_4$ alkyl), where T is -O-, -S-, -SO-, or -$SO_2$;
where

each G is independently a bond or $C_1$-$C_4$ alkylidene;

X is -H, or $C_1$-$C_4$ alkyl;

---- represents a single or double bond;

$R_a$ and $R_{a'}$ are independently hydrogen or $C_1$-$C_3$ alkyl, or when taken together with the carbon atom to which they are attached form a $C_3$-$C_8$ cycloalkyl ring;

p is 0, 1, or 2;

A is -O-, -S-, -NH-, or -$NCH_3$-; and

m is 0, 1, 2, or 3;

or a pharmaceutically-acceptable salt thereof, in the manufacture of a medicament for selectively antagonizing the 5-$HT_1$ receptor in a mammal.

**Patentansprüche**

**Patentansprüche für folgende Vertragsstaaten : AT, BE, CH, DE, FR, GB, IT, LI, LU, NL, SE**

**1.** Verwendung einer Verbindung der Formel

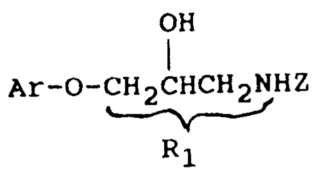

I ,

worin

Ar

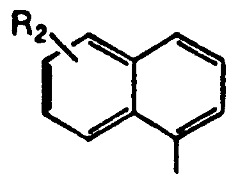 , 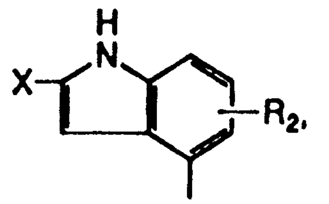 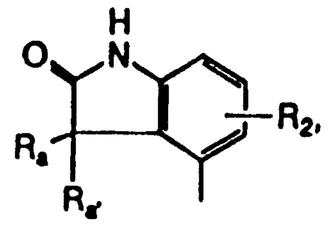

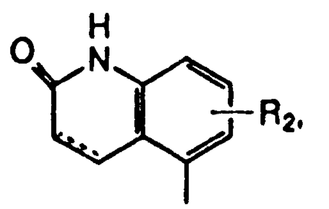 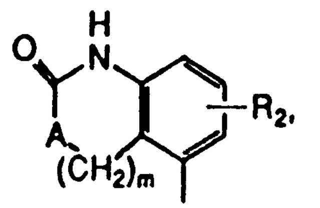 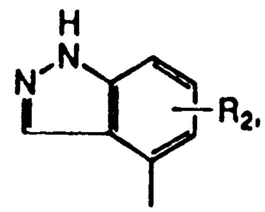

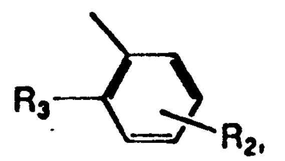 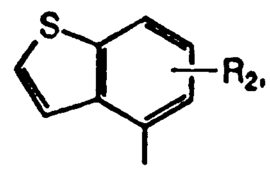

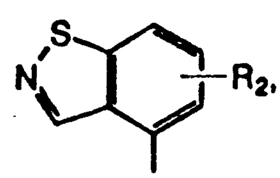 oder 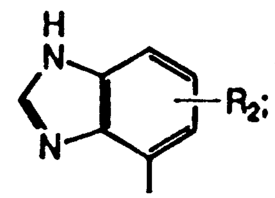

ist,

$R_1$ eine gegebenenfalls an einem der drei verbindenden Kohlenstoffatome substituierte Methylgruppe ist;

$R_2$ Wasserstoff, ein $C_1$-$C_4$-Alkyl-, Trifluormethyl-, Hydroxy-, ($C_1$-$C_4$-Alkyl)-O-, ($C_1$-$C_4$-Alkyl)-S(O)$_p$- oder Halogenrest ist;

$R_3$ ein $C_3$-$C_8$-Cycloalkylrest oder eine Bicycloalkylgruppe der Formel

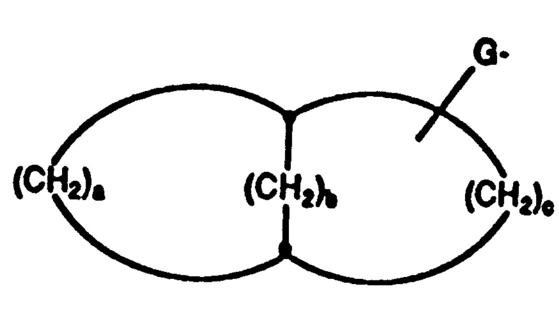

ist, worin a und c unabhängig 1 bis 5 sind, b 0 bis 5 ist und (a + c) größer als 2 ist;

Z eine geradkettige oder verzweigte $C_4$-$C_{10}$-Alkan-, Alken- oder Alkingruppe, eine gegebenenfalls mit $C_1$-$C_4$-Alkyl- oder Phenylresten substituierte ($C_4$-$C_8$-Cycloalkyl)-G-Gruppe, eine Bicycloalkylgruppe der Formel

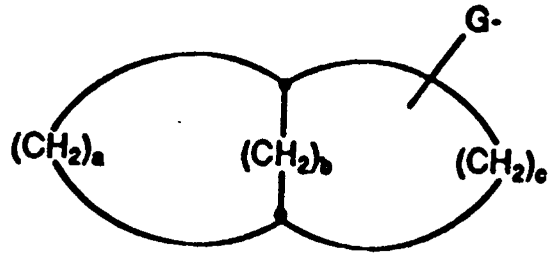 ,

worin a und c unabhängig 1 bis 5 sind, b 0 bis 5 ist und (a + c) größer als 2 ist, ein gegebenenfalls mit Phenylresten substituierter $C_2$-$C_{10}$-Alkylrest, bei dem die Phenylgruppe gegebenenfalls mit $R_2$, wie vorher definiert, substituiert sein kann, oder ein -($C_1$-$C_4$-Alkyliden)-T-($C_1$-$C_4$-alkyl)rest ist, worin T -O-, -S-, -SO- oder $SO_2$- ist;
worin
jedes G unabhängig eine Bindung oder ein $C_1$-$C_4$-Alkylidenrest ist;
X -H oder ein $C_1$-$C_4$-Alkylrest ist;
---- eine Einzel- oder Doppelbindung bedeutet;
$R_a$ und $R_{a'}$ unabhängig Wasserstoff oder $C_1$-$C_3$-Alkylreste sind oder zusammen mit dem Kohlenstoffatom, an das sie gebunden sind, einen $C_3$-$C_8$-Cycloalkylring bilden;
p 0, 1 oder 2 ist;
A -O-, -S-, -NH- oder -$NCH_3$- ist und
m 0, 1, 2 oder 3 ist;

oder eines pharmazeutisch annehmbaren Salzes davon zur Herstellung eines Arzneimittels, um den 5-$HT_1$-Rezeptor bei einem Säugetier selektiv zu antagonisieren.

**2.** Verwendung nach Anspruch 1, wobei eine Verbindung angewendet wird, worin Ar ein 1-Naphthyl-, 4-Indolyl- oder 2-Cyclopentylphenylrest ist.

**3.** Verwendung nach Anspruch 1, wobei eine Verbindung angewendet wird, worin Z ein Cyclohexyl-, Cycloheptyl-, Cyclooctyl- oder $C_6$-$C_8$-Alkylrest ist.

**4.** Verwendung nach Anspruch 2, wobei 1-(2-Cyclopentylphenoxy)-3-(cycloheptylamino)-2-propanol oder ein pharmazeutisch annehmbares Salz davon angewendet wird.

**5.** Verwendung nach Anspruch 2, wobei 1-(2-Cyclopentylphenoxy)-3-(cyclooctylamino)-2-propanol oder ein pharmazeutisch annehmbares Salz davon angewendet wird.

**6.** Verbindung der Formel Ia

$$\mathrm{Ar{-}O{-}CH_2\underset{\displaystyle |\atop \displaystyle OH}{CH}CH_2NHZ_a} \qquad \mathrm{Ia} \quad ,$$

worin

$Z_a$ ein ($C_6$-$C_8$-Cycloalkyl)-G-, $C_6$-$C_{10}$-Alkyl-, phenylsubstituierter $C_2$-$C_{10}$-Alkyl-, -($C_1$-$C_4$-Alkyliden)-T-($C_1$-$C_4$-alkyl)-rest oder

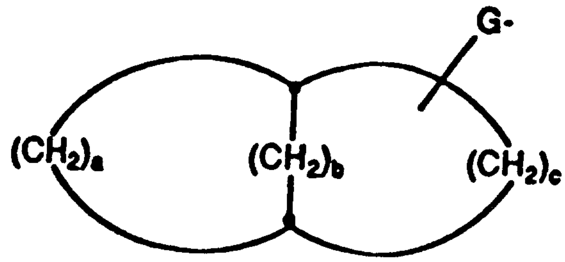

ist und

Ar

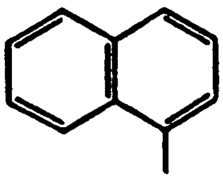

oder

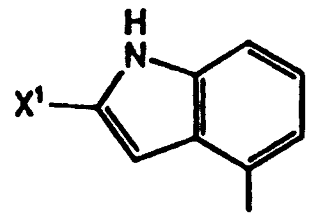

ist, worin

$X^1$ -H oder -CN ist;

jedes G unabhängig eine Bindung oder ein $C_1$-$C_4$-Alkylidenrest ist;

T -O-, -S-, -SO- oder -$SO_2$- ist und

und a und c unabhängig 1 bis 5 sind, b 0 bis 5 ist und (a + c) größer als 2 ist;

und pharmazeutisch annehmbare Salze davon, vorausgesetzt, daß dann,

a) wenn Ar ein 1-Naphthylrest ist, $Z_a$ kein Cyclohexyl-, $C_6$-$C_{10}$-Alkyl-, phenylsubstituierter $C_2$-$C_{10}$-Alkyl- oder -($C_1$-$C_4$-Alkyliden)T-($C_1$-$C_4$-alkyl)-rest sein kann und

b) wenn Ar

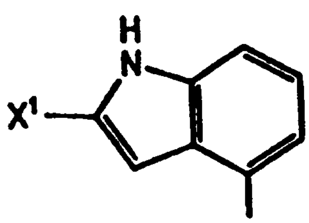

ist, $Z_a$ kein phenylsubstituierter $C_2$-$C_{10}$-Alkyl-, -($C_1$-$C_4$-Alkyliden)-T-($C_1$-$C_4$-alkyl)-rest oder

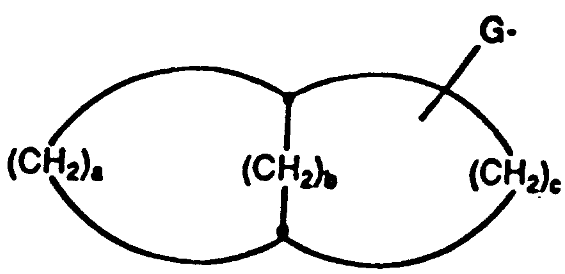

sein kann.

7. Pharmazeutische Zusammensetzung, umfassend eine Verbindung nach Anspruch 6 zusammen mit einem oder mehreren pharmazeutisch annehmbaren Hilfsstoffen, Verdünnungsmitteln oder Trägern.

8. Verfahren zur Herstellung einer Verbindung nach Anspruch 6, umfassend, daß man ein Epoxid der Formel

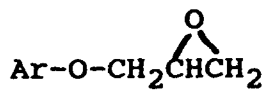

mit einem Amin der Formel $H_2NZ_a$ umsetzt und gegebenenfalls das entstehende Produkt in ein pharmazeutisch annehmbares Salz umwandelt.

**Patentansprüche für folgende Vertragsstaaten : ES, GR**

1. Verfahren zur Herstellung einer Verbindung der Formel Ia

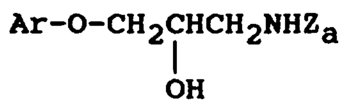

Ia ,

worin

$Z_a$ ein ($C_6$-$C_8$-Cycloalkyl)-G-, $C_6$-$C_{10}$-Alkyl-, phenylsubstituierter $C_2$-$C_{10}$-Alkyl-, -($C_1$-$C_4$-Alkyliden)-T-($C_1$-$C_4$-alkyl)-rest oder

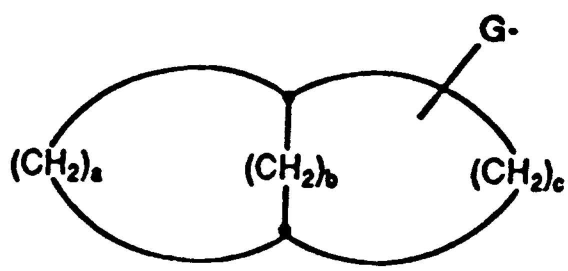

ist und

Ar

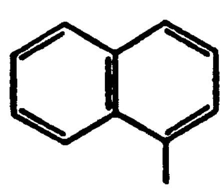

oder

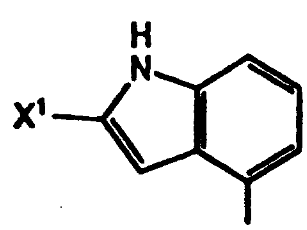

ist, worin

$X^1$ -H oder -CN ist;

jedes G unabhängig eine Bindung oder ein $C_1$-$C_4$-Alkylidenrest ist;

T -O-, -S-, -SO- oder -$SO_2$- ist und

und a und c unabhängig 1 bis 5 sind, b 0 bis 5 ist und (a + c) größer als 2 ist;

und pharmazeutisch annehmbare Salze davon, vorausgesetzt, daß dann,

a) wenn Ar ein 1-Naphthylrest ist, $Z_a$ kein Cyclohexyl-, $C_6$-$C_{10}$-Alkyl-, phenylsubstituierter $C_2$-$C_{10}$-Alkyl- oder -($C_1$-$C_4$-Alkyliden)-T-($C_1$-$C_4$-alkyl)-rest sein kann und
b) wenn Ar

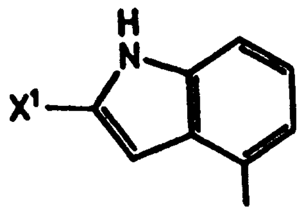

ist, $Z_a$ kein phenylsubstituierter $C_2$-$C_{10}$-Alkyl-, -($C_1$-$C_4$-Alkyliden)-T-($C_1$-$C_4$-alkyl)-rest oder

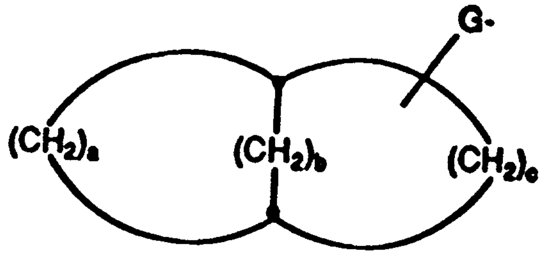

sein kann,
wobei das Verfahren umfaßt, daß man eine Epoxidverbindung der Formel

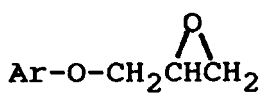

mit einem Amin der Formel $H_2NZ_a$ umsetzt und gegebenenfalls das entstehende Produkt in ein pharmazeutisch annehmbares Salz umwandelt.

**2.** Verfahren nach Anspruch 1 zur Herstellung einer Verbindung, worin Ar ein 1-Naphthyl-, 4-Indolyl- oder 2-Cyclo-4-indolylrest ist.

**3.** Verfahren nach Anspruch 1 zur Herstellung einer Verbindung, worin $Z_a$ ein Cyclohexyl-, Cycloheptyl-, Cyclooctyl- oder $C_6$-$C_8$-Alkylrest ist.

**4.** Verfahren zur Herstellung eines pharmazeutischen Präparates, umfassend, daß man eine Verbindung der Formel Ia, wie in einem der Ansprüche 1 bis 3 definiert, oder ein pharmazeutisch annehmbares Salz davon mit einem oder mehreren pharmazeutisch annehmbaren Trägern, Hilfsstoffen oder Verdünnungsmitteln vermischt.

**5.** Verwendung einer Verbindung der Formel

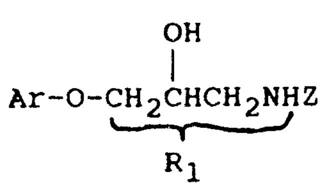

I ,

worin

Ar

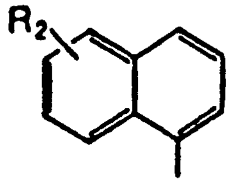 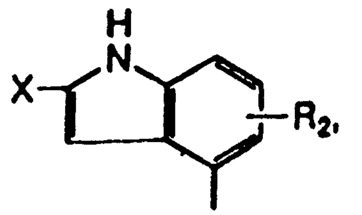 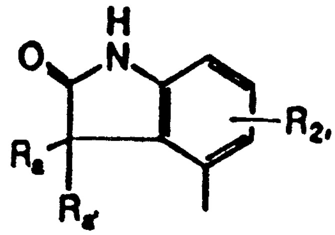

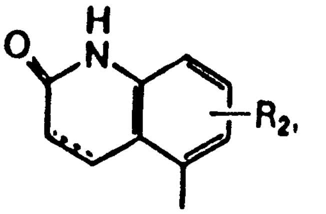 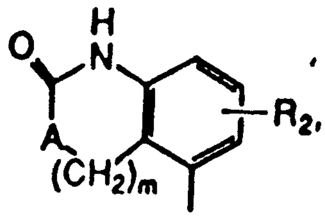 , 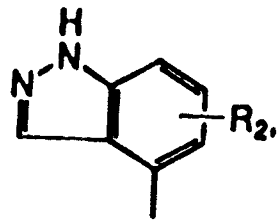

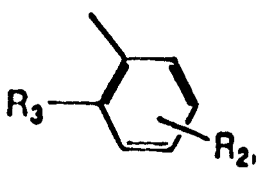 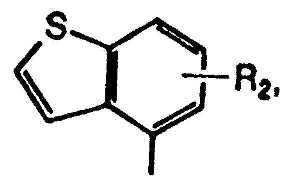

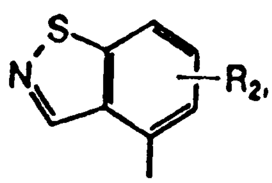 oder 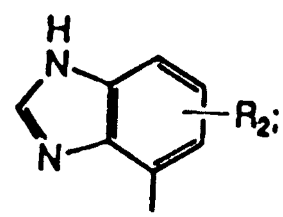

ist,

$R_1$ eine gegebenenfalls an einem der drei verbindenden Kohlenstoffatome substituierte Methylgruppe ist;

$R_2$ Wasserstoff, ein $C_1$-$C_4$-Alkyl-, Trifluormethyl-, Hydroxy-, ($C_1$-$C_4$-Alkyl)-O-, ($C_1$-$C_4$-Alkyl)-S(O)$_p$- oder Halogenrest ist;

$R_3$ ein $C_3$-$C_8$-Cycloalkylrest oder eine Bicycloalkylgruppe der Formel

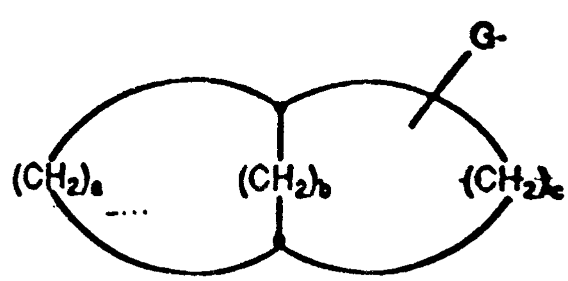

ist, worin a und c unabhängig 1 bis 5 sind, b 0 bis 5 ist und (a + c) größer als 2 ist;

Z eine geradkettige oder verzweigte $C_4$-$C_{10}$-Alkan-, Alken- oder Alkingruppe, eine gegebenenfalls mit $C_1$-$C_4$-Alkyl- oder Phenylresten substituierte ($C_4$-$C_8$-Cycloalkyl)-G-Gruppe, eine Bicycloalkylgruppe der Formel

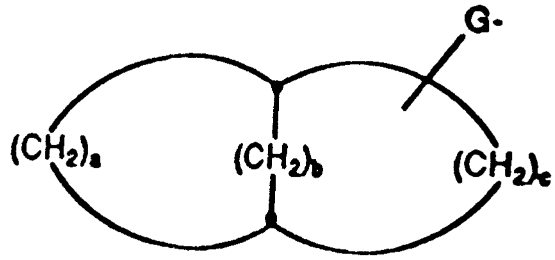,

worin a und c unabhängig 1 bis 5 sind, b 0 bis 5 ist und (a + c) größer als 2 ist, ein gegebenenfalls mit Phenylresten substituierter $C_2$-$C_{10}$-Alkylrest, bei dem die Phenylgruppe gegebenenfalls mit $R_2$, wie vorher definiert, substituiert sein kann, oder ein -($C_1$-$C_4$-Alkyliden)-T-($C_1$-$C_4$-alkylrest) ist, worin T -O-, -S-, -SO- oder -$SO_2$- ist;

worin

jedes G unabhängig eine Bindung oder ein $C_1$-$C_4$-Alkylidenrest ist;

X -H oder ein $C_1$-$C_4$-Alkylrest ist;

---- eine Einzel- oder Doppelbindung bedeutet;

$R_a$ und $R_{a'}$ unabhängig Wasserstoff oder $C_1$-$C_3$-Alkylreste sind oder zusammen mit dem Kohlenstoffatom, an das sie gebunden sind, einen $C_3$-$C_8$-Cycloalkylring bilden;

p 0, 1 oder 2 ist;

A -O-, -S-, -NH- oder -$NCH_3$- ist und

m 0, 1, 2 oder 3 ist;

oder eines pharmazeutisch annehmbaren Salzes davon zur Herstellung eines Arzneimittels, um den 5-$HT_1$-Rezeptor bei einem Säugetier selektiv zu antagonisieren.

**Revendications**

**Revendications pour les Etats contractants suivants : AT, BE, CH, DE, FR, GB, IT, LI, LU, NL, SE**

**1.** Utilisation d'un composé répondant à la Formule

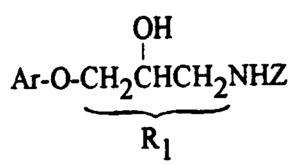

I

dans laquelle Ar représente

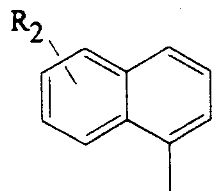
,
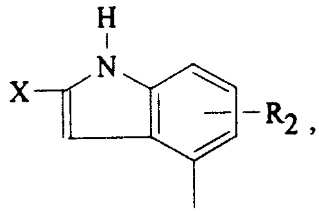
,
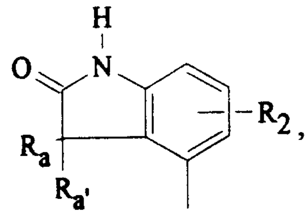
,
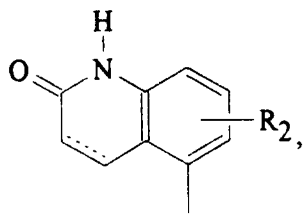
,
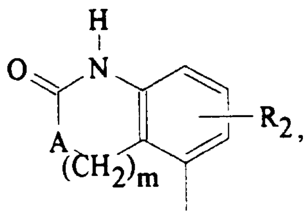
,
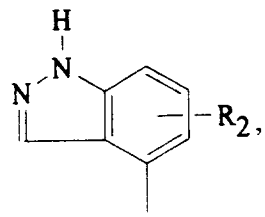
,
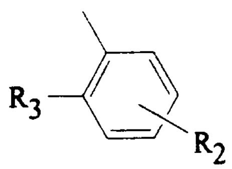
,
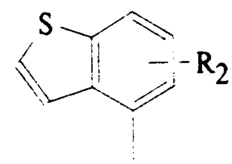
,
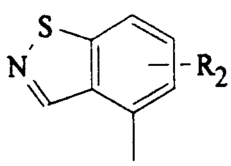
ou
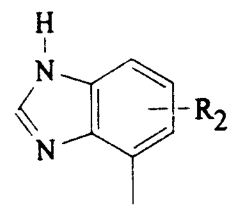
;

$R_1$ représente un groupe méthyle facultatif substitué sur un des trois atomes de carbone de liaison;
$R_2$ représente un atome d'hydrogène, un groupe alkyle en $C_1$-$C_4$, un groupe trifluorométhyle, un groupe hydroxyle, un groupe (alkyle en $C_1$-$C_4$)-O-, un groupe (alkyle en $C_1$-$C_4$)-$S(O)_p$-, ou un atome d'halogène;
$R_3$ représente un groupe cycloalkyle en $C_3$-$C_8$ ou un groupe bicycloalkyle répondant à la formule

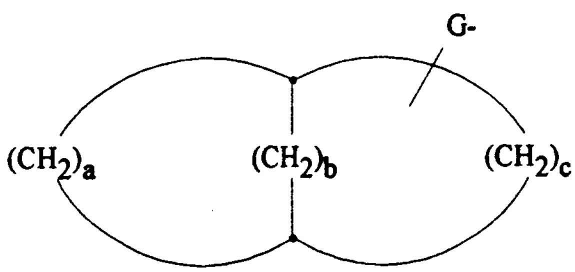
,

où a et c valent, indépendamment l'un de l'autre, 1-5, b vaut 0-5, et (a + c) est supérieur à 2;
Z représente un groupe alcane, alcène ou alcyne en $C_4$-$C_{10}$ linéaire ou ramifié, un groupe (cycloalkyle en $C_4$-$C_8$)-G- facultativement substitué par un groupe alkyle en $C_1$-$C_4$ ou par un groupe phényle, un groupe bicycloalkyle répondant à la formule

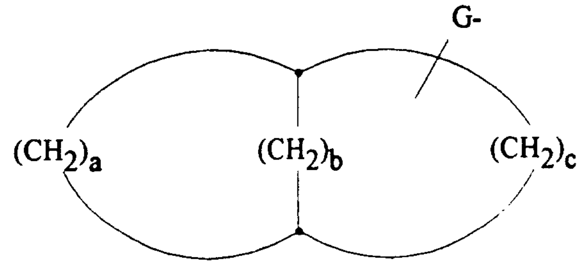

,

dans laquelle a et c valent, indépendamment l'un de l'autre, 1-5, b vaut 0-5, et (a + c) est supérieur à 2, facultativement un alkyle en $C_2$-$C_{10}$ substitué par un groupe phényle, où le groupe phényle peut être facultativement substitué avec $R_2$ tel qu'il a été défini précédemment, ou un groupe -(alkylidène en $C_1$-$C_4$)-T-(alkyle en $C_1$-$C_4$), où T représente -O-, -S-, -SO-, ou -$SO_2$-;
où

chaque G représente, indépendamment l'un de l'autre, une liaison ou un alkylidène en $C_1$-$C_4$;
X représente -H ou un groupe alkyle en $C_1$-$C_4$;
----- représente une simple liaison ou une double liaison;
$R_a$ et $R_{a'}$ représentent, indépendamment l'un de l'autre, un atome d'hydrogène ou un groupe alkyle en $C_1$-$C_3$, ou lorsqu'ils sont pris ensemble avec l'atome de carbone auquel ils sont liés forment un noyau cycloalkyle en $C_3$-$C_8$;
p vaut 0, 1 ou 2;
A représente -O-, -S-, -NH- ou -$NCH_3$-; et
m vaut 0, 1, 2 ou 3;

ou un sel pharmaceutiquement acceptable de ceux-ci, dans la préparation d'un médicament pour agir comme antagoniste sélectif du récepteur 5-$HT_1$ chez un mammifère.

2. Utilisation selon la revendication 1, employant un composé dans lequel Ar représente un groupe 1-naphtyle, un groupe 4-indolyle ou un groupe 2-cyclopentylphényle.

3. Utilisation selon la revendication 1, employant un composé dans lequel Z représente un groupe cyclohexyle, un groupe cycloheptyle, un groupe cyclooctyle ou un groupe alkyle en $C_6$-$C_8$.

4. Utilisation selon la revendication 2, employant le 1-(2-cyclopentylphénoxy)-3-(cycloheptylamino)-2-propanol ou un sel pharmaceutiquement acceptable de celui-ci.

5. Utilisation selon la revendication 2, employant le 1-(2-cyclopentylphénoxy)-3-(cyclooctylamino)-2-propanol ou un sel pharmaceutiquement acceptable de celui-ci.

6. Composé répondant à la Formule Ia

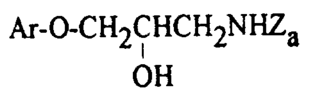

Ia

où $Z_a$ représente un groupe (cycloalkyle en $C_6$-$C_8$)-G-, un groupe alkyle en $C_6$-$C_{10}$, un groupe alkyle en $C_2$-$C_{10}$ substitué par un groupe phényle, un groupe -(alkylidène en $C_1$-$C_4$)-T-(alkyle en $C_1$-$C_4$) ou

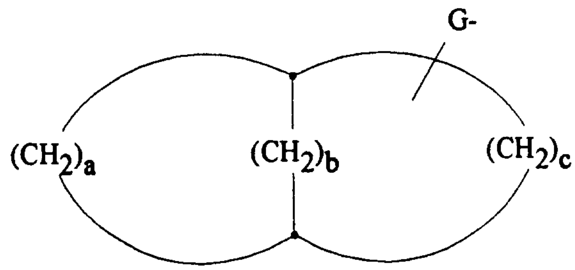

et Ar représente

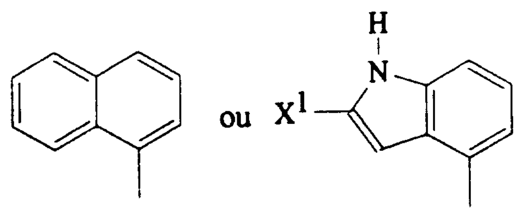

où

$X^1$ représente -H ou -CN;

chaque G représente, indépendamment l'un de l'autre, une liaison ou un groupe alkylidène en $C_1$-$C_4$;

T représente -O-, -S-, -SO- ou -$SO_2$-; et

a et c valent, indépendamment l'un de l'autre, 1-5, b vaut 0-5 et (a + c) est supérieur à 2;

et les sels pharmaceutiquement acceptables de ceux-ci, pour autant que

a) lorsque Ar représente un groupe 1-naphtyle, $Z_a$ ne peut pas représenter un groupe cyclohexyle, un groupe alkyle en $C_6$-$C_{10}$, un groupe alkyle en $C_2$-$C_{10}$ substitué par un groupe phényle ou un groupe -(alkylidène en $C_1$-$C_4$)-T-(alkyle en $C_1$-$C_4$); et

b) lorsque Ar représente

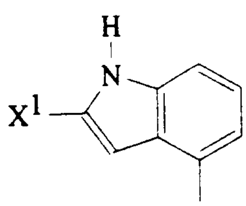

$Z_a$ ne peut pas représenter un groupe alkyle en $C_2$-$C_{10}$ substitué par un groupe phényle, un groupe (alkylidène en $C_1$-$C_4$)-T-(alkyle en $C_1$-$C_4$) ou

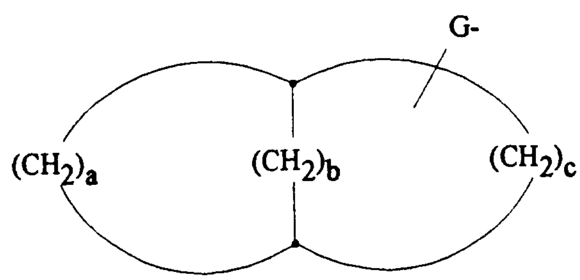

7. Composition pharmaceutique comprenant un composé selon la revendication 6, en association avec un ou plusieurs excipients, diluants ou supports pharmaceutiquement acceptables pour celui-ci.

8. Procédé pour la préparation d'un composé selon la revendication 6, qui comprend la mise en réaction d'un composé époxyde répondant à la formule

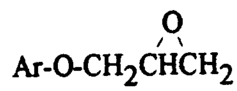

avec une amine répondant à la formule $H_2NZ_a$ et facultativement la conversion du produit résultant en un sel pharmaceutiquement acceptable.

**Revendications pour les Etats contractants suivants : ES, GR**

1. Procédé pour la préparation d'un composé répondant à la Formule Ia

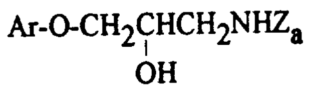

Ia

où $Z_a$ représente un groupe (cycloalkyle en ($C_6$-$C_8$)-G-, un groupe alkyle en $C_6$-$C_{10}$, un groupe alkyle en $C_2$-$C_{10}$ substitué par un groupe phényle, un groupe (alkylidène en $C_1$-$C_4$)-T-(alkyle en $C_1$-$C_4$) ou

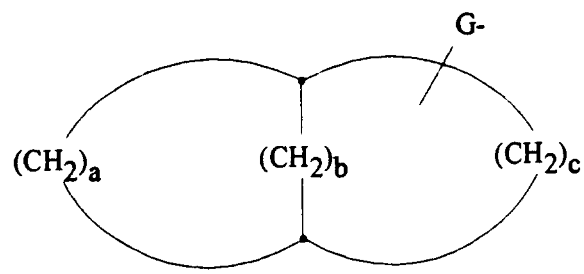

et Ar représente

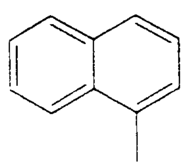

ou

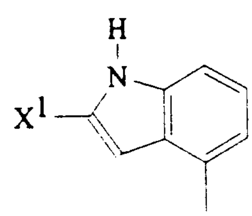

où

$X^1$ représente -H ou -CN;

chaque G représente, indépendamment l'un de l'autre, une liaison ou un groupe alkylidène en $C_1$-$C_4$;

T représente -O-, -S-, -SO- ou -$SO_2$-; et

a et c valent, indépendamment l'un de l'autre, 1-5, b vaut 0-5 et (a + c) est supérieur à 2;

et les sels pharmaceutiquement acceptables de ceux-ci, pour autant que

a) lorsque Ar représente un groupe 1-naphtyle, $Z_a$ ne peut pas représenter un groupe cyclohexyle, un groupe alkyle en $C_6$-$C_{10}$, un groupe alkyle en $C_2$-$C_{10}$ substitué par un groupe phényle ou un groupe -(alkylidène en $C_1$-$C_4$)-T-(alkyle en $C_1$-$C_4$); et

b) lorsque Ar représente

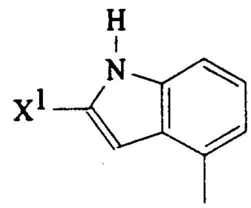

$Z_a$ ne peut pas représenter un groupe alkyle en $C_2$-$C_{10}$ substitué par un groupe phényle, un groupe (alkylidène en $C_1$-$C_4$)-T-(alkyle en $C_1$-$C_4$) ou

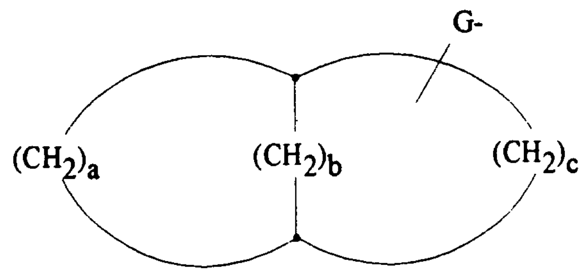

;

lequel procédé comprend la mise en réaction d'un composé époxyde répondant à la formule

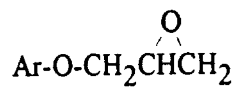

avec une amine répondant à la formule $H_2NZ_a$ et facultativement la conversion du produit résultant en un sel pharmaceutiquement acceptable.

**2.** Procédé selon la revendication 1 pour la préparation d'un composé dans lequel Ar représente un groupe 1-naphtyle, un groupe 4-indolyle ou un groupe 2-cyano-4-indolyle.

**3.** Procédé selon la revendication 1 pour la préparation d'un composé dans lequel $Z_a$ représente un groupe cyclohexyle, un groupe cycloheptyle, un groupe cyclooctyle ou un groupe alkyle en $C_6$-$C_8$.

**4.** Procédé pour la préparation d'une formulation pharmaceutique qui comprend le mélange d'un composé répondant à la Formule Ia tel que défini dans l'une quelconque des revendications 1 à 3, ou un sel pharmaceutiquement acceptable de celui-ci, avec un ou plusieurs supports, excipients ou diluants pharmaceutiquement acceptables pour celui-ci.

**5.** Utilisation d'un composé répondant à la Formule

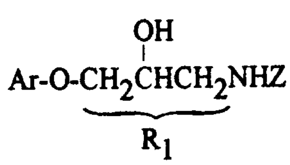

I

dans laquelle Ar représente

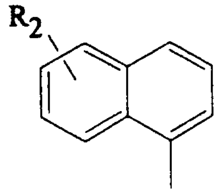 , 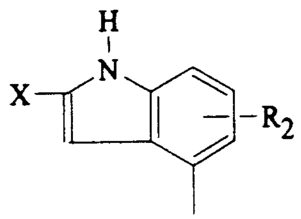 , 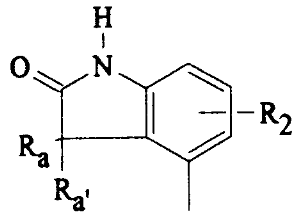 ,

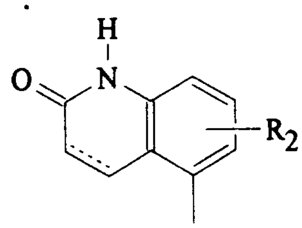 , 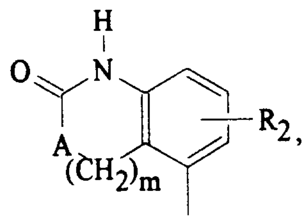 , 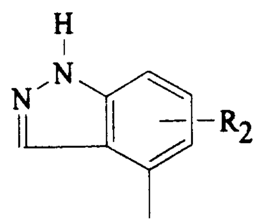 ,

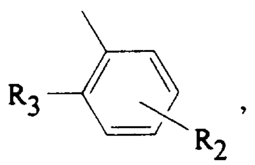 , 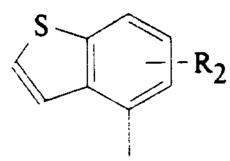 ,

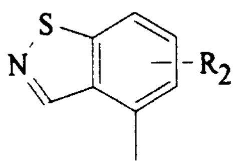 ou 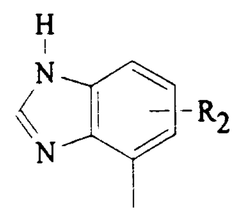 ;

$R_1$ représente un groupe méthyle facultatif substitué sur un des trois atomes de carbone de liaison;
$R_2$ représente un atome d'hydrogène, un groupe alkyle en $C_1$-$C_4$, un groupe trifluorométhyle, un groupe hydroxyle, un groupe (alkyle en $C_1$-$C_4$)-O-, un groupe (alkyle en $C_1$-$C_4$)-$S(O)_p$-, ou un atome d'halogène;
$R_3$ représente un groupe cycloalkyle en $C_3$-$C_8$ ou un groupe bicycloalkyle répondant à la formule

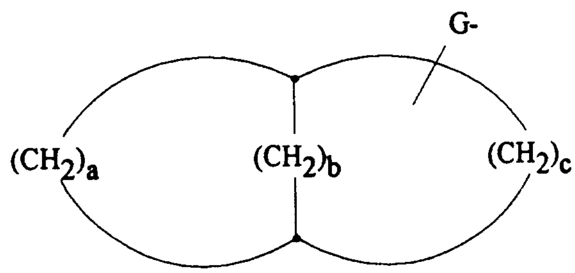

où a et c valent, indépendamment l'un de l'autre, 1-5, b vaut 0-5, et (a + c) est supérieur à 2;
Z représente un groupe alcane, alcène ou alcyne a $C_4$-$C_{10}$ linéaire ou ramifié, un groupe (cycloalkyle en $C_4$-$C_8$)-G- facultativement substitué par un groupe alkyle en $C_1$-$C_4$ ou un groupe phényle, un groupe bicycloalkyle répondant à la formule

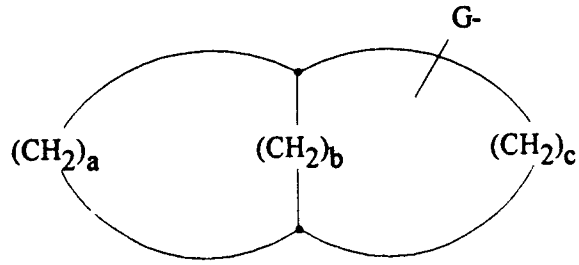

,

dans laquelle a et c valent, indépendamment l'un de l'autre, 1-5, b vaut 0-5, et (a + c) est supérieur à 2, facultativement un alkyle en $C_2$-$C_{10}$ substitué par un groupe phényle, où le groupe phényle peut être facultativement substitué avec R tel qu'il a été défini précédemment, ou un groupe -(alkylidène en $C_1$-$C_4$)-T-(alkyle en $C_1$-$C_4$), où T représente O-, -S-,-SO-, ou -$SO_2$-;
où

chaque G représente, indépendamment l'un de l'autre, une liaison ou un alkylidène en $C_1$-$C_4$;

X représente -H ou un groupe alkyle en $C_1$-$C_4$;

----- représente une simple liaison ou une double liaison;

$R_a$ et $R_{a'}$ représentent, indépendamment l'un de l'autre, un atome d'hydrogène ou un groupe alkyle en $C_1$-$C_3$, ou lorsqu'ils sont pris ensemble avec l'atome de carbone auquel ils sont liés forment un noyau cycloalkyle en $C_3$-$C_8$;

p vaut 0, 1 ou 2;

A représente -O-, -S-, -NH- ou -$NCH_3$-; et

m vaut 0, 1, 2 ou 3;

ou un sel pharmaceutiquement acceptable de ceux-ci, dans la préparation d'un médicament pour agir comme antagoniste sélectif du récepteur 5-$HT_1$ chez un mammifère.